# EUROPÄISCHE PATENTSCHRIFT

(11) **EP 3 459 470 B1**
(45) Veröffentlichungstag und Bekanntmachung des Hinweises auf die Patenterteilung: **04.09.2019**
(21) Anmeldenummer: 17192869.0
(22) Anmeldetag: 25.09.2017
(51) Int. Cl.: A61B 17/122, A61B 17/02, A61B 17/128, A61B 17/00

(54) **PLASTISCH VERFORMBARER CHIRURGISCHER CLIP**
PLASTICALLY DEFORMABLE SURGICAL CLIP
CLIP CHIRURGICAL PLASTIQUEMENT DÉFORMABLE

(43) Veröffentlichungstag der Anmeldung: 27.03.2019
(73) Patentinhaber: Lazic Besitz GmbH & Co. KG, 78532 Tuttlingen (DE)
(72) Erfinder: Lazic, Daniel, 78532 Tuttlingen (DE)
(74) Vertreter: Kohler Schmid Möbus Patentanwälte

(56) Entgegenhaltungen:
- WO-A1-97/11645
- DE-A1- 3 443 367
- DE-A1-102011 001 798
- US-A- 4 671 281
- US-A1- 2012 184 976

## Beschreibung

Die Erfindung betrifft einen einstückigen, chirurgischen Clip, insbesondere aus Metall, mit zwei Klemmarmen und mit einem die beiden Klemmarme miteinander einstückig verbindenden, nicht-federelastischen Verbindungsabschnitt, wobei die beiden Klemmarme durch plastische Verformung des Clips aus einer geöffneten Ausgangsstellung aufeinanderzu bis in eine geschlossene Endstellung schwenkbar sind, voneinander beabstandet an dem Verbindungsabschnitt angreifen und über den Verbindungsabschnitt hinaus jeweils durch einen angeformten Hebelarm verlängert sind und wobei durch Zusammendrücken der beiden Hebelarme die beiden Klemmarme durch plastische Verformung des Verbindungsabschnitts aus der geschlossenen Endstellung in eine geöffnete Stellung schwenkbar sind.

Ein derartiger chirurgischer Clip ist beispielsweise durch die DE 34 43 367 A1 bekannt geworden.

Solche chirurgischen Clips dienen allgemein zum Abklemmen von Gefäßen.

Der aus der DE 34 43 367 A1 bekannte chirurgische Clip dient insbesondere zur mikrovascularen Anastomose und verfügt über ein Paar gebogener Beine, die über einen Brückenabschnitt miteinander in Verbindung stehen. Der Clip ist aus einem einheitlichen Stück eines biologisch verträglichen, plastisch deformierbaren Materials hergestellt und verfügt über zwei im Abstand voneinander angeordnete Ohren zur Erleichterung seiner Handhabung und Entfernung des Clips. Zur Anbringung des Clips dient ein Werkzeug, das in einer pinzettenartigen Einrichtung besteht, die über zwei Arme zum Zusammendrücken des Clips verfügt.

Aus der US 4 671 281 A ist ein Clip 23 aus Polymermaterial mit zwei Hebelarmen und mit einem elastischen Gelenkbereich bekannt, wobei die Hebelarme mittels eines Keilelements dauerhaft aufgespreizt werden müssen, um den Clip zu schließen. Ein äußerer Verbindungsarm des Clips weist außerdem elastische Knickstellen auf, um den äußeren Verbindungsarm über einen Totpunkt hinweg in die Aufspreizposition zu bewegen.

Ein aus DE 195 20 158 A1 bekannter chirurgischer Clip ist aus Metall und U- oder V-förmig mit zwei Klemmarmen und mit einem diese beiden Klemmarme miteinander einstückig verbindenden Verbindungsteil ausgebildet, welches die Funktion eines plastisch verformbaren Scharniers hat. Die beiden Klemmarme tragen an der Innenseite des Clips einander zugewandte profilierte Klemmflächen, die bei plastischer Verformung des Verbindungsabschnitts dauerhaft klemmend aneinander andrückbar sind. Der Clip wird durch Zusammenpressen der beiden Klemmarme kraftschlüssig geschlossen und kann nicht wieder geöffnet werden.

Die vorliegende Erfindung stellt sich demgegenüber die Aufgabe, einen chirurgischen Clip der eingangs genannten Art dahingehend weiterzubilden, dass er aus seiner geschlossenen Endstellung wieder geöffnet werden kann, um ihn wieder zu entfernen.

Diese Aufgabe wird erfindungsgemäß dadurch gelöst, dass die beiden Hebelarme, insbesondere an ihren äußeren Enden, miteinander durch einen äußeren Verbindungsarm verbunden sind, der durch Zusammendrücken der beiden Hebelarme plastisch verformbar ist. Durch den äußeren Verbindungsarm sind die beiden Cliphälften beim Zusammendrücken der Klemm- und Hebelarme stabilisiert. Bevorzugt ist der chirurgische Clip ein lasergeschnittener Zuschnitt aus Metall oder auch aus Kunststoff, bei dem der Verbindungsabschnitt mindestens zweimal plastisch verformt werden kann.

Die beiden Klemmarme sind durch plastische Verformung des Verbindungsabschnitts, des Übergangs zwischen Klemmarm und Verbindungsabschnitt oder einer Kombination davon aus der geöffneten Ausgangsstellung aufeinanderzu bis in die geschlossene Endstellung schwenkbar.

Um eine Sollknickstelle auszubilden, weist der Verbindungsabschnitt eine geringere Querschnittfläche als die Klemm- und/oder Hebelarme auf, also beispielsweise bei gleicher Dicke eine geringere Breite als die Klemm- und/oder Hebelarme.

Besonders bevorzugt liegen der von den Klemmarmen und den Hebelarmen jeweils eingeschlossene Außenwinkel zwischen 100° und 170°, bevorzugt zwischen 120° und 150°, und in der Ausgangsstellung der von den Klemmarmen und dem Verbindungsabschnitt jeweils eingeschlossene Innenwinkel zwischen 100° und 170°, bevorzugt zwischen 120° und 150°.

Vorzugsweise haben die beiden Hebelarme an ihren einander abgewandten Außenseiten jeweils einen Vorsprung, damit der Clip von einer Zange definiert gefasst werden kann.

Um eine Sollknickstelle auszubilden, weist der äußere Verbindungsabschnitt bevorzugt eine geringere Querschnittfläche als die Klemm- und/oder Hebelarme auf, also beispielsweise bei gleicher Dicke eine geringere Breite als die Klemm- und/oder Hebelarme. Der äußere Verbindungsarm ist vorteilhaft in der Ausgangsstellung, insbesondere V-förmig, nach außen abgewinkelt, um so beim Zusammendrücken der Hebelarme eine vordefinierte Sollknickstelle zu bilden.

Vorzugsweise weist der äußere Verbindungsarm einen abstehenden Fortsatz auf, an dem ein Faden oder Band angebracht oder ein (Silikon)Schlauch aufgesteckt werden kann, um Gewebe halten zu können, beispielsweise um die Hirnhaut bei einer Operation aus dem Weg spannen zu können.

Die Erfindung betrifft auch einen Cliphalter, insbesondere aus Metall, mit einer Halteplatte und mit mindestens einem, bevorzugt mit mehreren wie oben ausgebildeten, an der Halteplatte jeweils über mindestens eine Sollbruchstelle einstückig verbundenen chirurgischen Clips. Um Kosten zu sparen, werden die Clips zusammen mit der Halterplatte aus einem Blech gefertigt, insbesondere lasergeschnitten. Der Cliphalter bildet zugleich ein Bedienteil, das man gut in der Hand halten kann und von dem ein Clip mittels einer Zange abgedreht werden kann. Die Sollbruchstelle ist konstruktiv dünner als der Clip, so dass beim Abdrehen der Clip formstabil bleibt.

Vorzugsweise ist die Sollbruchstelle an der den Klemmarmen zugewandten Innenseite des Verbindungsabschnitts angeordnet, da hier eine scharfe Abbruchstelle nicht störend und somit die Verletzungsgefahr sehr gering ist.

Weitere Vorteile und vorteilhafte Ausgestaltungen des Gegenstands der Erfindung ergeben sich aus der Beschreibung, den Ansprüchen und den Zeichnungen. Ebenso können die vorstehend genannten und die noch weiter aufgeführten Merkmale je für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung. Es zeigen:
- Fign. 1a-1c: einen nicht erfindungsgemäßen, ersten chirurgischen Clip in einer geöffneten Ausgangsstellung (Fig. 1a), in einer geschlossenen Endstellung (Fig. 1b) und in einer wieder geöffneten Stellung (Fig. 1c);
- Fign. 2a-2c: einen zweiten erfindungsgemäßen chirurgischen Clip in der geöffneten Ausgangsstellung (Fig. 2a), in einer geschlossenen Endstellung (Fig. 2b) und in einer wieder geöffneten Stellung (Fig. 2c);
- Fign. 3a, 3b: den in Fig. 2 gezeigten zweiten chirurgischen Clip mit einem zusätzlichen Fortsatz (Fig. 3a) und mit einem darauf aufgesteckten Schlauch (Fig. 3b); und
- Fig. 4: einen Cliphalter mit mehreren daran einstückig angeformten erfindungsgemäßen chirurgischen Clips.

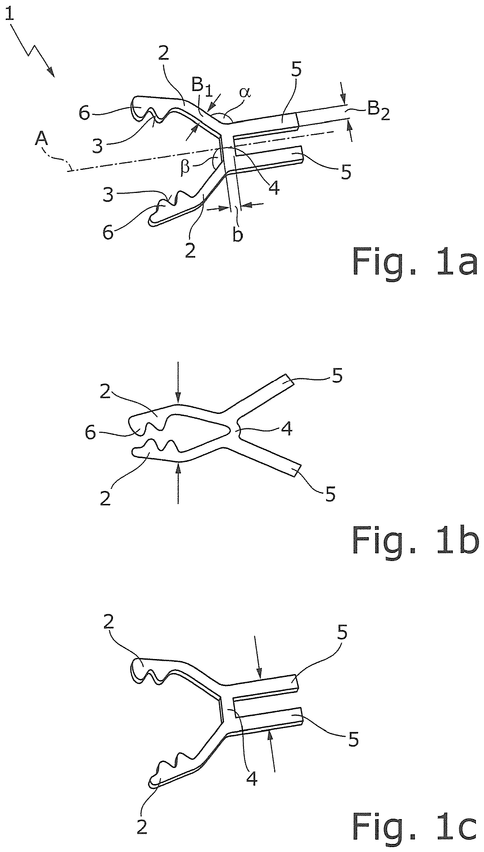

Der in **Fig. 1a** gezeigte einstückige chirurgische Clip **1** ist bezüglich seiner Längsmittelebene **A** spiegelsymmetrisch und umfasst zwei abgeknickte Klemmarme **2,** die an ihren einander zugewandten Innenseiten Klemmflächen **3** aufweisen, einen die beiden Klemmarme 2 miteinander einstückig verbindenden, nicht-federelastischen Verbindungsabschnitt **4,** an dem die beiden Klemmarme 2 voneinander beabstandet angreifen, und zwei an den Klemmarmen 2 einstückig angeformte Hebelarme **5,** die die beiden Klemmarme 2 über den Verbindungsabschnitt 4 hinaus verlängern und die in der in Fig. 1a gezeigten Ausgangsstellung parallel zueinander verlaufen. Die Klemm- und Hebelarme 2, 5 bilden also zwei Cliphälften, die durch den Verbindungsabschnitt 4 voneinander beabstandet sind.

Der Clip 1 hat eine einheitliche Dicke und kann beispielsweise ein lasergeschnittener Blech- oder Kunststoffzuschnitt sein. Die Breite **b** des Verbindungsabschnitts 4 ist kleiner als die Breiten **B1, B2** der Klemm- und Hebelarme 2, 5, so dass der Verbindungsabschnitt 4 aufgrund seiner geringeren Querschnittfläche eine Sollknickstelle ausbildet. Der von den Klemmarmen 2 und den Hebelarmen 5 jeweils eingeschlossene Außenwinkel **α** beträgt ca. 135°, und der von den Klemmarmen 2 und dem Verbindungsabschnitt 4 jeweils eingeschlossene Innenwinkel **β** beträgt in der in Fig. 1a gezeigten Ausgangsstellung ebenfalls ca. 135°.

Durch Zusammendrücken der beiden Klemmarme 2 mittels einer Zange, wie in **Fig. 1b** durch die beiden Pfeile angedeutet ist, können die beiden Klemmarme 2 durch plastische Verformung des Verbindungsabschnitts 4 aus der geöffneten Ausgangsstellung aufeinanderzu bis in eine geschlossene Endstellung geschwenkt bzw. gebogen werden, in der Zähne **6** der Klemmflächen 3 ineinandergreifen. Der Verbindungsabschnitt 4 hat somit die Funktion eines Scharniers. Beim Zusammendrücken werden der Klemmarm 2 und der daran angreifende Hebelarm 5 aufgrund ihrer größeren Querschnittfläche zueinander nicht verbogen, sondern bleiben formstabil.

Durch Zusammendrücken der beiden nun aufgespreizten Hebelarme 5 mittels einer Zange, wie in **Fig. 1c** durch die beiden Pfeile angedeutet ist, können die beiden Klemmarme 2 durch plastische Verformung des Verbindungsabschnitts 4 aus der geschlossenen Endstellung zurück in eine geöffnete Stellung geschwenkt bzw. gebogen werden. Beim Zusammendrücken werden der Klemmarm 2 und der daran angreifende Hebelarm 5 aufgrund ihrer größeren Querschnittfläche zueinander nicht verbogen, sondern bleiben formstabil.

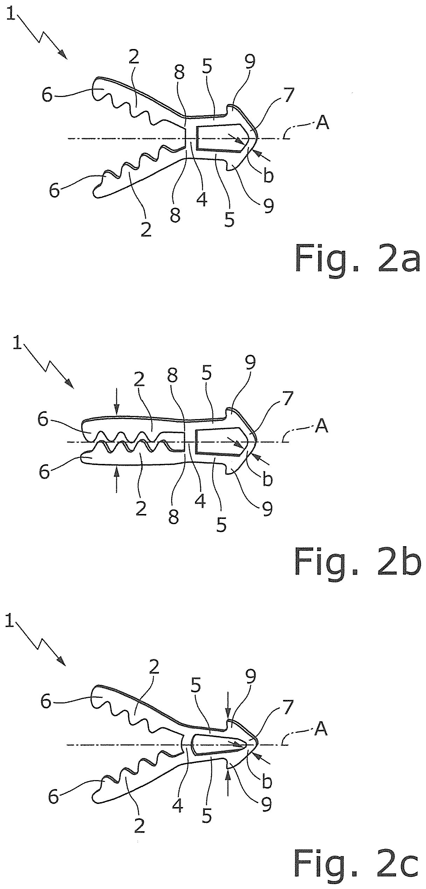

Vom Clip der Fig. 1 unterscheidet sich der in **Fig. 2a** gezeigte Clip 1 im Wesentlichen dadurch, dass hier die beiden Hebelarme 5 an ihren äußeren Enden miteinander durch einen äußeren Verbindungsarm **7** verbunden sind, der eine geringere Querschnittfläche als die Klemm- und Hebelarme 2, 5 aufweist. Der äußere Verbindungsarm 7 ist V-förmig nach außen abgewinkelt und weist die gleiche Dicke, aber eine geringere Breite b als die Breite der Klemm- und Hebelarme 2, 5 auf, um eine Sollknickstelle auszubilden. Statt abgewinkelt wie in Fig. 1 sind die Klemmarme 2 gebogen ausgeführt.

Durch Zusammendrücken der beiden Klemmarme 2 mittels einer Zange, wie in **Fig. 2b** durch die beiden Pfeile angedeutet ist, können die beiden Klemmarme 2 jeweils durch plastische Verformung des Übergangs **8** zwischen Klemmarm 2 und Verbindungsabschnitt 4 aus der geöffneten Ausgangsstellung aufeinanderzu bis in eine geschlossene Endstellung geschwenkt bzw. gebogen werden, in der die Zähne 6 der Klemmflächen 3 ineinandergreifen. Der Übergang 8 hat somit bei der Schließbewegung die Funktion eines Scharniers. Beim Zusammendrücken der Klemmarme 2 werden der Verbindungsabschnitt 4 und die Hebelarme 5 aufgrund des äußeren Verbindungsarms 7 nicht plastisch verformt, sondern bleiben formstabil.

Durch Zusammendrücken der beiden Hebelarme 5 mittels einer Zange, wie in **Fig. 2c** durch die beiden Pfeile angedeutet ist, können die beiden Klemmarme 2 durch plastische Verformung des Verbindungsabschnitts 4 aus der geschlossenen Endstellung zurück in eine geöffnete Stellung geschwenkt bzw. gebogen werden. Beim Zusammendrücken werden der Klemmarm 2 und der daran angreifende Hebelarm 5 aufgrund ihrer größeren Querschnittfläche zueinander nicht verbogen, sondern bleiben formstabil. Beim Zusammendrücken der Hebelarme 5 wird auch der äußere Verbindungsarm 7 plastisch verformt und verhindert, dass die beiden Cliphälften aus der Clipebene ausbrechen. Weiterhin weisen die beiden Hebelarme 5 endseitig an ihren einander abgewandten Außenseiten jeweils einen Vorsprung **9** auf, um den Clip 1 in einer Zange besser aufnehmen zu können.

Die beiden Klemmarme 2 sind bei dem Clip 1 der Fig. 1 durch plastische Verformung des Verbindungsabschnitts 4 und bei dem Clip 1 der Fig. 2 durch plastische Verformung des Übergangs 8 aufeinanderzu bis in die geschlossene Endstellung schwenkbar, können hier bei nicht gezeigten Clips aber auch durch eine kombinierte plastische Verformung des Verbindungsabschnitts 4 und des Übergangs 8 in die geschlossene Endstellung verschwenkt werden.

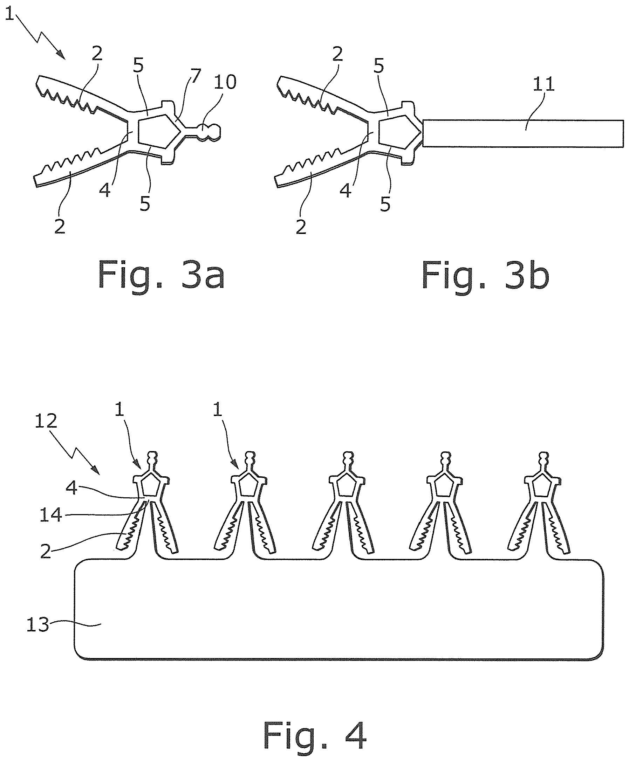

**Fig. 3a** zeigt den Clip 1 von Fig. 2 mit einem zusätzlichen Fortsatz **10,** der am äußeren Verbindungsarm 7, genauer gesagt an der Spitze des V-förmigen äußeren Verbindungsarms 7, einstückig angeformt ist und nach hinten absteht. Am Fortsatz 10 kann ein Faden angebracht oder, wie in **Fig. 3b** gezeigt, ein Silikonschlauch **11** aufgesteckt werden, um Gewebe halten zu können, beispielsweise um die Hirnhaut bei einer Operation aus dem Weg spannen zu können.

Der in **Fig. 4** gezeigte Cliphalter **12** umfasst eine Halteplatte **13** und mehrere, an der Halteplatte 13 jeweils über eine Sollbruchstelle **14** einstückig verbundene Clips 1. Die Clips 1 sind zusammen mit der Halteplatte 12 aus einem Blech gefertigt, insbesondere aus einem Metallblech lasergeschnitten. Die Sollbruchstelle 14 befindet sich an der den Klemmarmen 2 zugewandten Innenseite des Verbindungsabschnitts 4, da hier eine scharfe Abbruchstelle nicht störend und die Verletzungsgefahr sehr gering ist. Die Sollbruchstelle 14 ist konstruktiv dünner als der Rest des Clips 1, so dass beim Abdrehen der Clip 1 formstabil bleibt. Der Cliphalter 12 bildet zugleich ein Bedienteil, das man gut in der Hand halten kann. Der Bediener kann mit der anderen Hand die beiden Klemmarme 2 eines Clips 1 mittels einer Zange fassen, den Clip 1 durch Abdrehen der Sollbruchstelle 14 von der Halteplatte 13 lösen und den Clip 1 dann, ohne umgreifen oder neu fassen zu müssen, direkt applizieren.

## Patentansprüche

1. Einstückiger chirurgischer Clip (1), insbesondere aus Metall, mit zwei Klemmarmen (2) und mit einem die beiden Klemmarme (2) miteinander einstückig verbindenden, nicht-federelastischen Verbindungsabschnitt (4), wobei die beiden Klemmarme (2) durch plastische Verformung des Clips (1) aus einer geöffneten Ausgangsstellung aufeinanderzu bis in eine geschlossene Endstellung schwenkbar sind, voneinander beabstandet an dem Verbindungsabschnitt (4) angreifen und über den Verbindungsabschnitt (4) hinaus jeweils durch einen angeformten Hebelarm (5) verlängert sind und wobei durch Zusammendrücken der beiden Hebelarme (5) die beiden Klemmarme (2) durch plastische Verformung des Verbindungsabschnitts (4) aus der geschlossenen Endstellung in eine geöffnete Stellung schwenkbar sind,
**dadurch gekennzeichnet,**
**dass** die beiden Hebelarme (5), insbesondere an ihren äußeren Enden, miteinander durch einen äußeren Verbindungsarm (7) verbunden sind, der durch Zusammendrücken der beiden Hebelarme (5) plastisch verformbar ist.

2. Chirurgischer Clip nach Anspruch 1, **dadurch gekennzeichnet, dass** die beiden Klemmarme (2) durch plastische Verformung des Verbindungsabschnitts (4) aus der geöffneten Ausgangsstellung aufeinanderzu bis in die geschlossene Endstellung schwenkbar sind.

3. Chirurgischer Clip nach Anspruch 1 oder 2, **dadurch gekennzeichnet, dass** die beiden Klemmarme (2) jeweils durch plastische Verformung des Übergangs (8) zwischen Klemmarm (2) und Verbindungsabschnitt (4) aus der geöffneten Ausgangsstellung aufeinanderzu bis in die geschlossene Endstellung schwenkbar sind.

4. Chirurgischer Clip nach einem der vorhergehenden Ansprüche, **dadurch gekennzeichnet, dass** der Verbindungsabschnitt (4) eine geringere Querschnittfläche als die Klemm- und/oder Hebelarme (2, 5) aufweist.

5. Chirurgischer Clip nach Anspruch 4, **dadurch gekennzeichnet, dass** die Klemm- und/oder Hebelarme (2, 5) und der Verbindungsabschnitt (4) die gleiche Dicke aufweisen und die Breite (b) des Verbindungsabschnitts (4) kleiner als die Breite der Klemm- und/oder Hebelarme (2, 5) ist.

6. Chirurgischer Clip nach einem der vorhergehenden Ansprüche, **dadurch gekennzeichnet, dass** die beiden Hebelarme (5) an ihren einander abgewandten Außenseiten jeweils einen Vorsprung (9) aufweisen.

7. Chirurgischer Clip nach einem der vorhergehenden Ansprüche, **dadurch gekennzeichnet, dass** der äußere Verbindungsarm (7) eine geringere Querschnittfläche als die Klemm- und Hebelarme (2, 5) aufweist.

8. Chirurgischer Clip nach Anspruch 7, **dadurch gekennzeichnet, dass** die Klemm- und/oder Hebelarme (2, 5) und der äußere Verbindungsarm (7) die gleiche Dicke aufweisen und die Breite (b) des äußeren Verbindungsarms (7) kleiner als die Breite der Klemm- und/oder Hebelarme (2, 5) ist.

9. Chirurgischer Clip nach einem der vorhergehenden Ansprüche, **dadurch gekennzeichnet, dass** zumindest in der geschlossenen Endstellung der äußere Verbindungsarm (7), insbesondere V-förmig, nach außen abgewinkelt ist.

10. Chirurgischer Clip nach einem der vorhergehenden Ansprüche, **dadurch gekennzeichnet, dass** der äußere Verbindungsarm (7) einen nach außen abstehenden Fortsatz (10) aufweist.

11. Chirurgischer Clip nach einem der vorhergehenden Ansprüche, **dadurch gekennzeichnet, dass** der chirurgische Clip (1), mit Ausnahme von an den Klemmarmen (2) vorhandenen Zähnen (6), bezüglich seiner Längsmittelebene (A) spiegelsymmetrisch ist.

12. Chirurgischer Clip nach einem der vorhergehenden Ansprüche, **dadurch gekennzeichnet, dass** der chirurgische Clip (1) ein lasergeschnittener Zuschnitt ist.

13. Cliphalter (12), insbesondere aus Metall, mit einer Halteplatte (13) und mit mindestens einem, bevorzugt mit mehreren, an der Halteplatte (13) jeweils über mindestens eine Sollbruchstelle (14) einstückig verbundenen chirurgischen Clip (1) nach einem der vorhergehenden Ansprüche.

14. Cliphalter nach Anspruch 13, **dadurch gekennzeichnet, dass** die Sollbruchstelle (14) an der den Klemmarmen (2) zugewandten Innenseite des Verbindungsabschnitts (4) angeordnet ist.

## Claims

1. One-piece surgical clip (1), in particular from metal, comprising two clamping arms (2) and a non-spring-elastic connection portion (4) that interconnects the two clamping arms (2) in an integral manner, wherein the two clamping arms (2) on account of a plastic deformation of the clip (1) are pivotable towards one another from an opened initial position up to a closed end position, engage on the connection portion (4) so as to be mutually spaced apart and are in each case extended beyond the connection portion (4) by one lever arm (5) moulded thereon, and wherein, by squeezing the two lever arms (5), the two clamping arms (2), on account of a plastic deformation of the connection portion (4), are pivotable from the closed end position to an opened position, **characterized in that** the two lever arms (5), in particular at the external ends thereof, are interconnected by an external connection arm (7), which can be plastically deformed by squeezing the two lever arms (5).

2. Surgical clip according to claim 1, **characterized in that** the two clamping arms (2) on account of a plastic deformation of the connection portion (4) are pivotable towards one another from the opened initial position up to the closed end position.

3. Surgical clip according to claim 1 or 2, **characterized in that** the two clamping arms (2) on account of a plastic deformation of the transition (8) between the clamping arm (2) and the connection portion (4) are in each case pivotable towards one another from the opened initial position up to the closed end position.

4. Surgical clip according to one of the preceding claims, **characterized in that** the connection portion (4) has a smaller cross-sectional area than the clamping arms (2) and/or lever arms (5).

5. Surgical clip according to claim 4, **characterized in that** the clamping arms (2) and/or lever arms (5) and the connection portion (4) have the same thickness, and the width (b) of the connection portion (4) is smaller than the width of the clamping arms (2) and/or lever arms (5).

6. Surgical clip according to one of the preceding claims, **characterized in that** the two lever arms (5) on external sides thereof that face away from one another have in each case one protrusion (9).

7. Surgical clip according to one of the preceding claims, **characterized in that** the external connection arm (7) has a smaller cross-sectional area than the clamping arms (2) and lever arms (5).

8. Surgical clip according to claim 7, **characterized in that** the clamping arms (2) and/or lever arms (5) and the external connection arm (7) have the same thickness, and the width (b) of the external connection arm (7) is smaller than the width of the clamping arms (2) and/or lever arms (5).

9. Surgical clip according to one of the preceding claims, **characterized in that** the external connection arm (7) at least in the closed end position is angled outwards, in particular in a V-shaped manner.

10. Surgical clip according to one of the preceding claims, **characterized in that** the external connection arm (7) has an outwardly projecting extension (10).

11. Surgical clip according to one of the preceding claims, **characterized in that** the surgical clip (1), with the exception of teeth (6) that are present on the clamping arms (2) is mirror-symmetrical in relation to the longitudinal central plane (A) of said surgical clip (1).

12. Surgical clip according to one of the preceding claims, **characterized in that** the surgical clip (1) is a laser-cut blank.

13. Clip holder (12), in particular from metal, comprising a holding plate (13) and at least one surgical clip (1), preferably a plurality of surgical clips (1), according to one of the preceding claims, which is in each case connected to the holding plate (13) in an integral manner by way of at least one predetermined breaking point (14).

14. Clip holder according to claim 13, **characterized in that** the predetermined breaking point (14) is disposed on the internal side of the connection portion (4) that faces the clamping arms (2).

## Revendications

1. Pince chirurgicale monobloc (1), notamment en métal, comprenant deux bras de pincement (2) et une zone de liaison (4) non douée d'élasticité, qui relie les deux bras de pincement (2) l'un à l'autre d'un seul tenant, sachant que les deux bras de pincement (2) peuvent être animés de pivotements en direction l'un de l'autre par déformation plastique de ladite pince (1), d'une position initiale ouverte jusqu'à une position extrême fermée, fusionnent dans la zone de liaison (4) avec espacement l'un de l'autre et sont respectivement prolongés par un bras de levier (5) attenant, au-delà de ladite zone de liaison (4), et sachant que des pivotements peuvent être imprimés aux deux bras de pincement (2) vers une position ouverte, à partir de la position extrême fermée, par déformation plastique de ladite zone de liaison (4) et par compression des deux bras de levier (5),
**caractérisée par le fait**
**que** les deux bras de levier (5) sont reliés l'un à l'autre, notamment au niveau de leurs extrémités extérieures, par l'intermédiaire d'un bras extérieur de liaison (7) pouvant être déformé plastiquement par compression desdits deux bras de levier (5).

2. Pince chirurgicale selon la revendication 1, **caractérisée par le fait que** les deux bras de pincement (2) peuvent être animés de pivotements en direction l'un de l'autre, de la position initiale ouverte jusqu'à la position extrême fermée, par déformation plastique de la zone de liaison (4).

3. Pince chirurgicale selon la revendication 1 ou 2, **caractérisée par le fait que** les deux bras de pincement (2) peuvent être respectivement animés de pivotements en direction l'un de l'autre, de la position initiale ouverte jusqu'à la position extrême fermée, par déformation plastique de la transition (8) entre un bras de pincement (2) et la zone de liaison (4).

4. Pince chirurgicale selon l'une des revendications précédentes, **caractérisée par le fait que** la zone de liaison (4) présente une aire de section transversale moindre que celle(s) des bras de pincement et/ou des bras de levier (2, 5).

5. Pince chirurgicale selon la revendication 4, **caractérisée par le fait que** les bras de pincement et/ou les bras de levier (2, 5), et la zone de liaison (4), présentent la même épaisseur, et la largeur (b) de ladite zone de liaison (4) est inférieure à la largeur desdits bras de pincement et/ou bras de levier (2, 5).

6. Pince chirurgicale selon l'une des revendications précédentes, **caractérisée par le fait que** les deux bras de levier (5) sont respectivement munis d'une saillie (9) sur leurs faces extérieures pointant à l'opposé l'une de l'autre.

7. Pince chirurgicale selon l'une des revendications précédentes, **caractérisée par le fait que** le bras extérieur de liaison (7) présente une aire de section transversale moindre que celles des bras de pincement et des bras de levier (2, 5).

8. Pince chirurgicale selon la revendication 7, **caractérisée par le fait que** les bras de pincement et/ou les bras de levier (2, 5), et le bras extérieur de liaison (7), présentent la même épaisseur, et la largeur (b) dudit bras extérieur de liaison (7) est inférieure à la largeur desdits bras de pincement et/ou bras de levier (2, 5).

9. Pince chirurgicale selon l'une des revendications précédentes, **caractérisée par le fait que** le bras extérieur de liaison (7) est coudé vers l'extérieur, notamment avec configuration en V, au moins dans la position extrême fermée.

10. Pince chirurgicale selon l'une des revendications précédentes, **caractérisée par le fait que** le bras extérieur de liaison (7) est pourvu d'un appendice (10) dépassant vers l'extérieur.

11. Pince chirurgicale selon l'une des revendications précédentes, **caractérisée par le fait que** ladite pince chirurgicale (1) offre une symétrie spéculaire par rapport à son plan médian longitudinal (A), à l'exception de dents (6) présentes sur les bras de pincement (2).

12. Pince chirurgicale selon l'une des revendications précédentes, **caractérisée par le fait que** ladite pince chirurgicale (1) est une pièce prédécoupée au laser.

13. Porte-pinces (12) notamment en métal, comprenant une platine de retenue (13) et au moins une, de préférence plusieurs pinces chirurgicales (1) conformes à l'une des revendications précédentes, reliées d'une seul tenant à ladite platine de retenue (13) par l'intermédiaire d'au moins un point respectif (14) de rupture par destination.

14. Porte-pinces selon la revendication 13, **caractérisé par le fait que** le point (14) de rupture par destination est disposé à la face intérieure de la zone de liaison (4), qui est tournée vers les bras de pincement (2).
